Europäisches Patentamt

European Patent Office

Office européen des brevets

(19)

(11) Veröffentlichungsnummer: **0 483 645 B1**

# (12) EUROPÄISCHE PATENTSCHRIFT

(45) Veröffentlichungstag der Patentschrift: **14.06.95**

(51) Int. Cl.⁶: **C07C  51/265**, C07C 51/31, C07C 63/16

(21) Anmeldenummer: **91117988.5**

(22) Anmeldetag: **22.10.91**

(54) **Verfahren zur Erzeugung homogener o-Xylol-Naphthalin-Luft-Gemische für die Phthalsäureanhydrid-Herstellung.**

(30) Priorität: **02.11.90 DE 4034787**

(43) Veröffentlichungstag der Anmeldung:
**06.05.92 Patentblatt  92/19**

(45) Bekanntmachung des Hinweises auf die
Patenterteilung:
**14.06.95 Patentblatt  95/24**

(84) Benannte Vertragsstaaten:
**DE FR IT**

(56) Entgegenhaltungen:
**DE-A- 3 719 476**
**DE-C- 1 793 453**
**DE-C- 2 839 831**
**GB-A- 2 104 066**
**US-A- 2 329 638**

(73) Patentinhaber: **METALLGESELLSCHAFT AG**
**Postfach 10 15 01,**
**Reuterweg 14**
**D-60015 Frankfurt (DE)**

(72) Erfinder: **Saffran, Helmut**
**Porthstrasse 11**
**W-6000 Frankfurt/M. 50 (DE)**
Erfinder: **Keunecke, Gerhard**
**Sintherner Strasse 2**
**W-5024 Pulheim 5 (DE)**

EP 0 483 645 B1

**Beschreibung**

Die Erfindung betrifft ein Verfahren zur Erzeugung homogener o-Xylol-Naphthalin-Luft-Gemische für die katalytische Gasphasenoxidation zu Phthalsäureanhydrid.

Phthalsäureanhydrid wird technisch durch Luftoxidation von o-Xylol und/oder Naphthalin hergestellt (H. Suter, Phthalsäureanhydrid und seine Verwendung, Darmstadt 1972), wobei die Mischoxidation höhere Ausbeuten ergibt (DE-A 3 719 476). Zur Vermeidung lokaler Überhitzungen im Festbett-Röhrenreaktor und hierdurch bedingter Katalysator-Schädigung muß das Einsatz-Kohlenwasserstoff-Luft-Gemisch eine konstante Zusammensetzung bei homogener Vermischung aufweisen.

Die Erzeugung homogener o-Xylol-Luft-Gemische, meist durch Eindüsen von flüssigem o-Xylol in einen vorgewärmten Luftstrom, ist im wesentlichen unproblematisch (DE-C 1 793 453). Eine Direkteindüsung von flüssigem Naphthalin in den Oxidationsluftstrom verbietet sich hingegen, da Naphthalin, insbesondere solches aus Steinkohlenteer, Verunreinigungen enthält, die bei der Direkteinspritzung auf den Katalysator gelangen und dessen Aktivität und Lebensdauer vermindern würden.

Naphthalin wird daher getrennt verdampft, um auf diese Weise die unerwünschten Verunreinigungen abzutrennen. Hierzu wird ein Teilstrom der Oxidationsluft in flüssiges Naphthalin eingeleitet, so daß ein mit Naphthalin gesättigter Luftstrom entsteht, der danach mit der restlichen Oxidationsluft vermischt wird. Im Fall der Mischoxidation wird anschließend flüssiges o-Xylol in das Naphthalin-Luft-Gemisch eingespritzt (BE-A 893 521). Bei dieser Verfahrensweise wird das Naphthalin ständig erhöhter Temperatur in Anwesenheit von Luftsauerstoff ausgesetzt. Dies führt erfahrungsgemäß zu erhöhter Rückstandsbildung, so daß nicht nur Rückstand abzuziehen ist, sondern der Verdampfer in relativ kurzen Zeitintervallen gereinigt werden muß, was zu Kapazitäts- und Ausbeuteverlusten in Verbindung mit Umweltbelastung durch Reinigungsprozeduren führt. Auch die Direktverdampfung in Abwesenheit von Sauerstoff (DE-C 2 839 831) führt wegen der erforderlichen hohen Temperaturen zu vermehrter Rückstandsbildung.

In einem älteren Patent (US-A 2 329 638) wird vorgeschlagen, diese Rückstandsbildung durch Verdampfung des Naphthalins unter turbulenten Strömungsbedingungen in Gegenwart etwa der gleichen Gewichtsmenge Wasserdampf und nachträgliches Vermischen mit der Oxidationsluft zurückzudrängen. Derartige Mengen Wasserdampf führen jedoch zu erhöhter Phthalsäure-Bildung und folglich zu Ausbeute-Verlusten und zur Entstehung pyrophorer Eisensalze im Phthalsäureanhydrid-Abscheidesystem (H. Suter, Phthalsäureanhydrid und seine Verwendung, Darmstadt 1972, Seite 38 u. 61).

Ein neuerer Vorschlag (DE-A 3 719 476) sieht vor, bei Temperaturen unterhalb des Erstarrungspunktes von Naphthalin eine Lösung von Naphthalin in o-Xylol herzustellen und diese Lösung kurz vor der Reaktion auf 110 bis 180 °C zu erhitzen und in einen heißen Oxidationsluftstrom einzudüsen. Aufgrund der Löslichkeitsparameter ist dieses Verfahren jedoch auf Naphthalin-Konzentrationen von weniger als etwa 80 Gew.-%, vorzugsweise weniger als etwa 60 Gew.-% Prozent, beschränkt. Des weiteren muß das Naphthalin eine hohe Reinheit (Erstarrungspunkt 78 bis 80 °C) aufweisen, d. h. im Normalfall einer zusätzlichen, aufwendigen Destillation unterworfen werden, da die im technischen Naphthalin üblicherweise enthaltenen Verunreinigungen - wie eingangs erwähnt - als Katalysatorgift wirken.

Der Erfindung liegt daher die Aufgabe zugrunde, ein Verfahren zur Erzeugung homogener o-Xylol-Naphthalin-Luft-Gemische zu schaffen, bei dem die vorstehend genannten Nachteile vermieden werden.

Diese Aufgabe wird erfindungsgemäß entsprechend den Angaben der Patentansprüche gelöst.

Das vorliegende Verfahren stellt keine besonderen Anforderungen an die Qualität der Ausgangsstoffe; so kann z. B. Naphthalin aus Steinkohlenteer mit einer Reinheit von etwa 95 % ohne zusätzliche Destillation eingesetzt werden.

Bei dem erfindungsgemäßen Verfahren wird in Abwesenheit von molekularem Sauerstoff zunächst in bekannter Weise o-Xylol direkt verdampft, der o-Xylol-Dampf dann durch einen Naphthalin-Verdampfer bekannter Bauart geführt, wobei eine Sättigung mit Naphthalin-Dampf erfolgt, und das so erhaltene o-Xylol-Naphthalin-Gemisch mit dem Oxidationsluftstrom vermischt. Im allgemeinen wird eine Beladung im Bereich von 40 bis 100 g Kohlenwasserstoffe pro $Nm^3$ Luft angewendet.

Wie bei bekannten Verfahren auf o-Xylol-Basis ist Führungsgröße für das Rohstoff-Luftgemisch die Luftmenge, die ihrerseits durch die vorgegebene Leistung des Oxidationsreaktors bestimmt wird. Diese Luftmenge steuert die o-Xylol-Menge, die im gasförmigen oder flüssigen Zustand geregelt wird. Eine Regelung im flüssigen Zustand erfolgt dann, wenn die flüssige o-Xylol-Menge sofort und vollständig verdampft wird.

Die Sättigung des o-Xylol-Dampfes mit Naphthalin ist sowohl drucks- als auch temperaturabhängig. Die Temperatur-Regelung ist wegen des relativ großen Volumens an flüssigem Naphthalin träge und dient daher nur der Grundeinstellung. Die Feinregelung erfolgt durch Einstellung eines entsprechenden Verdampfungsdruckes, der über dem im Oxidationsreaktor herrschenden Druck liegen muß. Zweckmäßigerweise liegt der Verdampfungsdruck im Bereich von 1,1 bis 5,0 bar, vorzugsweise 1,3 bis 2,5 bar.

Die Zusammensetzung des dampfförmigen o-Xylol-Naphthalin-Gemisches entspricht folgender Gleichung:

$$\frac{G_{Naphthalin}}{G_{Xylol}} = \frac{MG_{Naphth.}}{MG_{Xylol}} \cdot \frac{P_{Naphth.}}{P_{Gesamt} - P_{Naphth.}}$$

mit:

$$P_{Naphth.} = \exp\left[A - \frac{B}{T + C}\right] \cdot \frac{1}{760} \qquad \text{(nach Antoine)}$$

wobei:

| | |
|---|---|
| G | = Gewicht Naphthalin bzw. o-Xylol |
| MG | = Molekulargewicht |
| $P_{Gesamt}$ | = Druck im Naphthalinverdampfer in bar bei der Temperatur T |
| $P_{Naphth.}$ | = Partialdruck des Naphthalins in bar bei der Temperatur T |
| T | = Temperatur im Naphthalinvedampfer in ° Kelvin |
| A | = Konstante = 16,1426 |
| B | = Konstante = 3992,01 |
| C | = Konstante = -71,29 |

(Antoine-Konstanten nach: Reid, Prausnitz u. Sherwood, The Properties of Gases and Liquids, New York, 1977)

Die Temperatur im Naphthalin-Vedampfer sollte einerseits möglichst niedrig liegen, um eine schonende Behandlung des Naphthalins sicherzustellen, andererseits ausreichen, um die gewünschten Kohlenwasserstoff-Mischungen herzustellen. Eine Temperatur im Bereich von 180 bis 220 °C, vorzugsweise bis 210 °C, erwies sich als optimal. Da der Grad der thermisch bedingten Rückstandsbildung in geringem Umfang auch von der Naphthalin-Qualität beeinflußt wird, kann im Einzelfall eine höhere Temperatur, z. B. 230 °C, zur Anwendung kommen.

Dieser Temperaturbereich entspricht bei einem bevorzugten Verdampfungsdruck von 1,3 bis 2,5 bar einer Naphthalin-Konzentration von etwa 20 bis 65 Gew.-%, bezogen auf o-Xylol-Naphthalin-Gemisch.

Sollte ein Rohstoff-Luft-Gemisch mit einem geringeren Naphthalin-Anteil gewünscht sein, so kann dies durch Erhöhen des Verdampfungsdruckes, gegebenenfalls unter geringfügiger Absenkung der Temperatur und/oder durch Verdünnen eines konzentrierteren o-Xylol-Naphthalin-Dampfgemisches mit der entsprechenden Menge o-Xylol erfolgen. Für die Verdünnung mit o-Xylol wird entweder ein Teilstrom des dampfförmigen o-Xylols im By-pass um den Naphthalin-Verdampfer herumgeführt oder flüssiges o-Xylol nach dem Naphthalin-Verdampfer in das Dampfgemisch bzw. in die Oxidationsluft eingespritzt.

Höhere Naphthalin-Konzentrationen lassen sich grundsätzlich durch Erhöhen der Temperatur im Naphthalin-Verdampfer erzielen, was jedoch mit einer verstärkten Rückstandsbildung einhergehen kann.

In einer speziellen Ausführung des erfindungsgemäßen Verfahrens werden daher zusätzlich zu dem o-Xylol-Dampf als Verdampfungshilfsmittel Wasserdampf, Kohlendioxid, Stickstoff, stickstoffhaltige Gase oder Gemische dieser in das flüssige Naphthalin eingeleitet. Bezogen auf die Dampfphase werden die anteiligen Volumina Naphthalin, o-Xylol und Verdampfungshilfsmittel durch deren Partialdrücke bestimmt, die anteiligen Gewichtsmengen zusätzlich durch die Molekulargewichte. Zur Erzielung eines bestimmten, höheren Naphthalin-Anteils im Rohstoff-Gemisch bei vorgegebener Maximal-Temperatur im Naphthalin-Verdampfer, von bevorzugt 210 °C, besonders bevorzugt 200 °C, braucht daher dem o-Xylol-Dampf nur eine im Vergleich zu der Kohlenwasserstoff-Menge geringe Menge an Verdampfungshilfsmittel entsprechend folgender Gleichung zugesetzt werden:

3

$$\frac{G_{Naphth.}}{G_{Xylol}} = \left[ \frac{MG_{Naphth.}}{MG_{Xylol}} + \frac{G_{Hilfsm.}}{G_{Xylol}} \cdot \frac{MG_{Naphth.}}{MG_{Hilfsm.}} \right] \cdot \frac{P_{Naphth.}}{P_{Ges.} - P_{Naphth.}}$$

wobei:

$G_{Hilfsm.}$ = Gewicht des Verdampfungshilfsmittels

$MG_{Hilfsm.}$ = Molekulargewicht des Verdampfungshilfsmittels

Wasserdampf wird wegen seiner gegenüber dem Naphthalin besonders guten Schutzwirkung und seines geringen Molekulargewichtes bevorzugt, sollte aber frei von mitgerissenen Salzen, insbesondere von Alkalisalzen sein, da andernfalls eine Beeinträchtigung des Oxidations-Katalysators auftreten könnte.

Aus vorstehender Gleichung ergibt sich, daß bei gleicher o-Xylol-Menge, gleicher Temperatur und gleichem Gesamtdruck ein Zusatz an Wasserdampf entsprechend etwa 17 % des Gewichtes an o-Xylol ausreicht, um gewichtsmäßig doppelt soviel Naphthalin zu verdampfen wie ohne Wasserdampf-Zusatz.

Die zusätzlich erforderlichen Wasserdampf-Mengen sind so gering, daß sie neben der mit der Oxidationsluft eingebrachten Feuchtigkeit und dem bei der Oxidation anfallenden Reaktionswasser während des weiteren Verlaufes des Phthalsäureanhydrid-Verfahrens nicht ins Gewicht fallen. So werden z. B.bei einem Gesamtdruck von 1,5 bar und einer Temperatur von 210 °C für 1.000 kg o-Xylol-Naphthalin-Dampfgemisch mit einem Naphthalin-Anteil von 85 Gew.-% nur 70 kg Wasserdampf benötigt. Die bei der nachfolgenden Oxidation zu Phthalsäureanhydrid entstehende Menge Reaktionswasser beträgt vergleichsweise etwa 580 kg pro 1.000 kg Kohlenwasserstoffe. Zusätzlich werden noch ca. 170 kg Wasser mit der Oxidationsluft (bei 20 °C und 60 % relative Feuchte) eingebracht.

Da die Verdampfung in Abwesenheit von molekularem Sauerstoff erfolgen sollte, kommen als Verdampfungshilfsmittel an sich nur sauerstofffreie Dämpfe oder Gase in Betracht. In Grenzfällen, insbesondere, wenn nur vereinzelt o-Xylol-Naphthalin-Dampf-Gemische mit geringfügig höheren Naphthalin-Konzentrationen zu erzeugen sind, kann auch Luft, sofern die erforderliche Menge gering ist, als Verdampfungshilfsmittel zugesetzt werden.

Die o-Xylol-Verdampfung erfolgt bevorzugt unter Verwendung eines Fallfilm-Verdampfers oder eines nach einem ähnlichen Prinzip konstruierten Verdampfers, der eine unmittelbare Verdampfung des zugeführten o-Xylols gewährleistet. Verdampfer mit einem Flüssigkeits-Sumpf sind wegen der längeren thermischen Belastung des Produktes eher ungeeignet.

Der Naphthalin-Verdampfer besteht vorzugsweise aus einem beheizten Kessel mit im oberen Dampfraum einer eingebauten Abstreifschicht, im darunter liegenden Dampfraum mehrerer Stoff-Austauschböden, z. B. Glockenböden, und im Sumpfraum einer frittenartigen, nach unten gerichteten Zufuhr für o-Xylol-Dampf und gegebenenfalls Verdampfungshilfsmittel. Ein mit der Verdampfer- Sumpfzone in Verbindung stehender Flüssig-Naphthalin-Kreislauf mit Erhitzer sorgt für Zufuhr der Verdampfungwärme des Naphthalins.

Das Naphthalin wird im geschmolzenen Zustand dem Verdampfer im oberen Bereich, in Höhe des obersten Stoff-Austauschbodens zugeführt und fließt unter ständiger Verdampfung langsam nach unten in die Sumpfzone, im Gegenstrom zu den o-Xylol-Dämpfen. Um eine Anreicherung von im Roh-Naphthalin stets enthaltenen schwere siedenden Komponenten in der Sumpfzone zu vermeiden, wird kontinuierlich ein geringer Anteil des Naphthalin-Sumpfes - je nach Qualität etwa 0,5 % bis 2 % - ausgeschleust.

Die aufsteigenden o-Xylol-Naphthalin-Dämpfe werden beim Durchtritt durch die Abstreifschicht von mitgerissenen Flüssigkeitsteilchen befreit und homogenisiert. Nach Austritt aus dem Verdampfer wird das Kohlenwasserstoff-Gemisch in einem geeigneten Gasmischer mit der vorgewärmten Oxidationsluft homogen vermischt und gelangt anschließend in einen Festbett-Röhrenreaktor zur Oxidation zu Phthalsäureanhydrid.

Das erfindungsgemäße Verfahren erlaubt die Erzeugung von o-Xylol-Naphthalin-Luft-Gemischen unter Vermeidung thermisch bzw. thermisch-oxidativ bedingter Rückstandsbildung im Naphthalin-Verdampfer. Eine zusätzliche Reinigung der technisch verfügbaren Rohstoffe ist nicht erforderlich. Die Konstanz der Zusammensetzung und die Homogenität der o-Xylol-Naphthalin-Luft-Gemische sind ausgezeichnet.

Beispiel 1:

Herzustellen ist ein o-Xylol-Naphthalin-Luft-Gemisch mit einem o-Xylol zu Naphthalin-Verhältnis von 60 : 40 Gewichts-Teilen und einer Beladung von 60 g Kohlenwasserstoffe pro $Nm^3$ Luft.

405 kg/h flüssiges Naphthalin (Reinheit 96 Gew.-%) werden mit einer Temperatur von 195 °C in den oberen Bereich eines Verdampfers eingeführt und gleichzeitig 600 kg/h o-Xylol-Dampf mit einer Temperatur von ebenfalls 195 °C in die Sumpfzone des Verdampfers eingeblasen. Zur Aufrechterhaltung einer Temperatur von 195 °C im Verdampfer werden ständig ca. 16.000 kg/h flüssiges Naphthalin der Sumpfzone entnommen und nach Erhitzen auf 200 °C wieder zugeführt. Des weiteren werden 5 kg/h Naphthalin aus dem Sumpfbereich ausgeschleust. Entsprechend dem gewünschten Naphthalin-Anteil wird der Verdampferdruck auf 1,62 bar eingestellt.

Dem aus dem Verdampfer austretenden Gemisch aus 127 Nm$^3$/h o-Xylol-Dampf und 70 Nm$^3$/h Naphthalin-Dampf werden mengengeregelt 16.667 Nm$^3$/h auf 150 °C vorgewärmte Oxidationsluft zugemischt. Anschließend wird dieses o-Xylol-Naphthalin-Gemisch in bekannter Weise zu Phthalsäureanhydrid umgesetzt.

Auch nach längerem Betreiben der Anlage wurden keinerlei Ablagerungen im Naphthalin-Verdampfer beobachtet. Die Mischoxidation verlief völlig problemlos, ohne Auftreten lokaler Temperaturspitzen.

Beispiel 2:

Herzustellen ist ein o-Xylol-Naphthalin-Luft-Gemisch mit einem o-Xylol zu Naphthalin-Verhältnis von 15 : 85 Gewichts-Teilen und einer Beladung von 65 g Kohlenwasserstoffe pro Nm$^3$ Luft.

Analog zu Beispiel 1 wird der Naphthalin-Verdampfer mit 860 kg/h flüssigem Naphthalin (Reinheit 96 Gew.-%) und 150 kg/h o-Xylol-Dampf bei einer Temperatur von 210 °C beschickt. Zusätzlich zu dem o-Xylol-Dampf werden jedoch 70 kg/h Wasserdampf (entmineralisiert) eingeblasen. Der Gesamt-Druck im Verdampfer wird auf 1,5 bar eingestellt. Die Menge an ausgeschleustem Naphthalin beträgt 10 kg/h.

Man erhält ein Gemisch aus 32 Nm$^3$/h o-Xylol-Dampf, 149 Nm$^3$/h Naphthalin-Dampf und 87 Nm$^3$/h Wasserdampf, dem vor Eintritt in den Oxidationsreaktor mengengeregelt 15.385 Nm$^3$/h Luft zugesetzt werden.

Ablagerungen im Naphthalin-Verdampfer wurden nicht festgestellt, ebensowenig lokale Überhitzungen im Oxidationsreaktor.

**Patentansprüche**

1. Verfahren zur Erzeugung homogener o-Xylol-Naphthalin-Luft-Gemische für die katalytische Gasphasenoxidation zu Phthalsäureanhydrid, dadurch gekennzeichnet, daß in Abwesenheit von molekularem Sauerstoff durch direkte Verdampfung erhaltener o-Xylol-Dampf durch Einleiten in flüssiges Naphthalin mit Naphthalin-Dampf gesättigt wird und das dampfförmige o-Xylol-Naphthalin-Gemisch mit vorgewärmter Oxidationsluft vermischt wird.

2. Verfahren gemäß Anspruch 1, dadurch gekennzeichnet, daß bei vorgegebenem o-Xylol-Naphthalin-Verhältnis die o-Xylol-Menge in Funktion der Oxidationsluft-Menge gesteuert wird, während die Naphthalin-Menge durch Einstellen von Temperatur und Druck des Naphthalin-Verdampfers geregelt wird.

3. Verfahren gemäß Anspruch 1, dadurch gekennzeichnet, daß zusätzlich zum o-Xylol-Dampf als Verdampfungshilfsmittel Wasserdampf, Kohlendioxid, Stickstoff, stickstoffhaltige Gase oder Gemische dieser in das flüssige Naphthalin eingeleitet werden.

4. Verfahren gemäß Anspruch 2 und 3, dadurch gekennzeichnet, daß bei vorgegebenem o-Xylol-Naphthalin-Verhältnis soviel Verdampfungshilfsmittel zugesetzt wird, daß die Temperatur im Naphthalin-Verdampfer 220 °C nicht überschreitet.

5. Verfahren gemäß Anspruch 4, dadurch gekennzeichnet, daß als Verdampfungshilfsmittel Wasserdampf zugesetzt wird und die Temperatur 210 °C nicht überschreitet.

**Claims**

1. A process of producing homogeneous mixtures of o-xylene, naphthalene and air for the gas-phase catalytic oxidation to form phthalic anhydride, characterized in that o-xylene vapour produced by a direct evaporation in the absence of molecular oxygen is saturated with naphthalene vapour by being introduced into liquid naphthalene and the vaporous mixture of o-xylene and naphthalene is mixed with preheated oxidizing air.

**2.** A process according to claim 1, characterized in that the ratio of o-xylene to naphthalene is predetermined and the rate of o-xylene is controlled as a function of the rate of oxidizing air whereas the rate of naphthalene is controlled by an adjustment of the temperature and pressure in the naphthalene evaporator.

**3.** A process according to claim 1, characterized in that water vapour, carbon dioxide, nitrogen, nitrogen-containing gases or mixtures thereof are introduced as an evaporation aid in addition to the o-xylene vapour into the liquid naphthalene.

**4.** A process according to claims 2 and 3, characterized in that the ratio of o-xylene to naphthalene is predetermined and the amount of evaporation aid is controlled so that the temperature in the naphthalene evaporator does not rise above 220 °C.

**5.** A process according to claim 4, characterized in that water vapor is added as an evaporation aid and temperature does not rise above 210 °C.

**Revendications**

**1.** Procédé de préparation de mélanges homogènes d'ortho-xylène, de naphtalène et d'air pour l'oxydation catalytique en phase gazeuse en anhydride phtalique, caractérisé en ce que, en l'absence d'oxygène moléculaire, on sature de vapeur de naphtalène, par introduction de naphtalène liquide, une vapeur d'ortho-xylène obtenue par évaporation directe, et on mélange de l'air d'oxydation préchauffé au mélange sous forme vapeur d'ortho-xylène et de naphtalène.

**2.** Procédé selon la revendication 1, caractérisé en ce que, en présence d'un rapport prédéfini de l'ortho-xylène au naphtalène, on ajuste le débit d'ortho-xylène en fonction du débit de l'air d'oxydation, tandis que le débit de naphtalène est régulé par ajustement de la température et de la pression de l'évaporateur de naphtalène.

**3.** Procédé selon la revendication 1, caractérisé en ce qu'on introduit dans le naphtalène liquide, outre la vapeur d'ortho-xylène servant d'auxiliaire d'évaporation, de la vapeur d'eau, de l'anhydride carbonique, de l'azote, des gaz azotés ou leurs mélanges.

**4.** Procédé selon les revendication 2 et 3, caractérisé en ce que, en présence d'un rapport prédéfini de l'ortho-xylène au naphtalène, on ajoute une quantité de l'auxiliaire d'évaporation suffisante pour que la température ne dépasse pas 220 °C dans l'évaporateur de naphtalène.

**5.** Procédé selon la revendication 4, caractérisé en ce qu'on ajoute de la vapeur d'eau en tant qu'auxiliaire d'évaporation, et que la température ne dépasse pas 210 °C.